# EUROPEAN PATENT APPLICATION

(11) **EP 4 122 453 A1**
(43) Date of publication of application: **25.01.2023**
(21) Application number: 21187147.0
(22) Date of filing: 22.07.2021
(51) Int. Cl.: A61K 9/51, A61K 9/19, A61K 31/58, A61L 26/00, A61P 1/04

(54) **THERMOREVERSIBLE GELATIN MATRIX FOR STABILITY ENHANCEMENT AND READY-TO-USE LIQUID APPLICATION OF NANOPARTICLES AND COLLOIDAL DRUGS**

(71) Applicant: Universität Heidelberg, 69117 Heidelberg (DE)
(72) Inventor: FRICKER, Gert, 69221 Dossenheim (DE); REICH, Gabriele, 69121 Heidelberg (DE); ERTHLE, Andreas, 69121 Heidelberg (DE); POLYAK, Fabian, 69190 Walldorf (DE)
(74) Representative: Müller-Boré & Partner Patentanwälte PartG mbB

(57) **Abstract**

The present invention relates to a novel thermoreversible sol-gel composition for stability enhancement and ready-to-use liquid application of nanoparticulate systems, a pharmaceutical composition, comprising said thermoreversible sol-gel composition, as well as an in-situ method of producing said thermoreversible sol-gel composition, wherein the nanoparticulate system comprises a biological polymer and/or a synthetic polymer. Furthermore, the present invention also relates to said thermoreversible sol-gel composition for use in medicine and specifically for use in treating *ulcerative colitis.*

## Description

The present invention relates to a novel thermoreversible sol-gel composition for stability enhancement and ready-to-use liquid application of nanoparticulate systems, a pharmaceutical composition comprising said thermoreversible sol-gel composition, as well as an in-situ method of producing said thermoreversible sol-gel composition, wherein the nanoparticulate system comprises a biological polymer and/or a synthetic polymer. Furthermore, the present invention also relates to said novel thermoreversible sol-gel composition for use in medicine and specifically for use in treating *ulcerative colitis.*

The development of new pharmaceutical products is increasingly confronted with the challenge of poorly water-soluble drug candidates. The solubility problem is often accompanied by a low intestinal permeability leading to a low bioavailability. Nanoparticulate systems, like liposomes and nanoparticles are one promising approach to improve the bioavailability of such drug candidates or to enhance the therapeutic effect of already marketed drug products (C. Medina et al., Br. J. Pharmacol., 2009, 150(5), 552-558).

Unfortunately, nanoparticulate systems show the general tendency to agglomerate over time leading to severe stability issues. All nanoparticulate systems tend to sediment over time, depending on the average particle size and the composition of the dispersion. This can ultimately lead to an irreversible aggregation, the so-called "caking". These considerable stability problems are based on the fact that nanoparticulate systems in aqueous dispersion generally form thermodynamically and energetically unfavorable systems (E. M. Hotze et al., J. Environ. Qual., 2010, 39(6), 1909). However, a sufficient long-term stability is essential for a technical or medical use of nanoparticulate systems.

One approach to stabilize nanoparticulate systems is the addition of surfactants to the dispersion medium. This addition can reduce, albeit not fully prevent, agglomeration and sedimentation of the nanoparticulate systems by electrostatic repulsion or steric hindrance. Another common strategy to improve stability is the deliberate increase of the viscosity of the dispersion medium in order to slow down particle sedimentation. Highly viscous liquids are however inherently more challenging to administer to a patient and thus can limit medical applications. Freeze-drying is the preferred procedure to stabilize colloidal drugs and carrier systems. The removal of the dispersion medium eliminates particle aggregation based on sedimentation within the freeze-dried product (N.-O. Chung et al., Int. J. Pharm., 2012, 437(1-2), 42-50).

However, the lyophilization process itself and the redispersion before application may have a negative impact on the drug carrier or the active pharmaceutical ingredient. This means that one has to take special care to avoid any increase in the average particle size or the loss of drug content (J. Han et al., Biomacromolecules, 2013, 14(5), 1529-1540; L. Niu et al., J. Controlled Release, 2017, 248, 125-132).

In addition, the drug product obtained by freeze-drying usually cannot be administered to a patient without additional preparation steps by medical staff, *i.e.* the lyophilizate must be redispersed prior to use. Furthermore, freeze drying is a technically complex production step associated with high costs and energy expenses. These drawbacks coupled with the already expensive production of nanoparticulate systems often prevent an economic realization of a pharmaceutical product. This might explain as to why promising colloidal formulations of drug candidates fail to reach market approval.

In view of the above, an object of the present invention is to provide a stable carrier system for nanoparticulate systems that enable an improved long-term storage while retaining the benefits of a liquid ready-to-use formulation at standard room temperature conditions.

In this context, the present invention provides a thermoreversible sol-gel composition for stability enhancement and ready-to-use liquid application of nanoparticulate systems, comprising: 0.5 to 15.0% by mass of a gelatin having a Bloom number of 60 to 325; a nanoparticulate system comprising at least one selected from the group consisting of a biological polymer, a synthetic polymer, a lipid, a metal core, and a silica core; a surfactant; and a biocompatible solvent; wherein the mass ratio [gelatin]/[surfactant(s)] is 0.5 to 10.0.

According to the present invention, a thermoreversible sol-gel composition represents a composition, which can be present (i) in form of a stable gel or (ii) in form of a liquid, e.g. a solution, a dispersion, or an emulsion, depending on the selected temperature.

In particular, according to the present invention, a thermoreversible sol-gel composition is provided, which exhibits a gel formation temperature of 2.0 to 10.0 °C, preferably in the range of 2.5 to 9.5 °C, more preferably 3.0 to 9.0 °C, most preferably 3.5 to 8.5 °C. For example, the gel formation temperature of the thermoreversible sol-gel composition is 2.0 to 9.5 °C, 2.0 to 9.0 °C, 2.0 to 8.5 °C, 2.5 to 10.0 °C, 2.5 to 9.0 °C, 2.5 to 8.5 °C, 3.0 to 10.0 °C, 3.0 to 9.5 °C, 3.0 to 8.5 °C, 3.5 to 10.0 °C, 3.5 to 9.5 °C, or 3.5 to 9.0 °C.

In addition, the thermoreversible sol-gel composition of the present invention exhibits a gel melting temperature of 15.0 to 25.0 °C, preferably in the range of 16.0 to 24.0 °C, more preferably 17.0 to 23.0 °C, most preferably 18.0 to 22.0 °C. For example, the gel melting temperature of the thermoreversible sol-gel composition is 15.0 to 24.0 °C, 15.0 to 23.0 °C, 15.0 to 22.0 °C, 16.0 to 25.0 °C, 16.0 to 23.0 °C, 16.0 to 22.0 °C, 17.0 to 25.0 °C, 17.0 to 24.0 °C, 17.0 to 22.0 °C, 18.0 to 25.0 °C, 18.0 to 24.0 °C, or 18.0 to 23.0 °C.

The gel formation temperature as well as the gel melting temperature of the thermoreversible sol-gel composition can be determined by low deformation oscillatory rheology. Herein, the crossover of the storage modulus G' and the loss modulus G" are used to define the gel formation temperature upon cooling and the gel melting temperature for the subsequent heating. Specifically, the measurement of the sol-gel transition of the thermoreversible sol-gel composition can be performed with a Haake MARS III^{™} rheometer (Thermo Scientific, Karlsruhe) using a parallel plate geometry with a diameter of 35 mm. In this context, the respective samples were freshly prepared 17 hours before use and stored at 10.0°C. Subsequently, the samples were gently melted before application to the rheometer and rim of the measuring gap enclosed with low-viscosity paraffin to avoid the loss of water. The sample was first cooled down to 4.0°C and subsequently heated at a rate of 1.0°C/min and the rheometer was operated in a controlled shear deformation mode with 1% deformation and a frequency of 1 Hz while recording the shear stress amplitude. The dynamic viscoelastic moduli G' (storage modulus) and G" (loss modulus) were evaluated as a function of the temperature.

According to the present invention, if the above-defined thermoreversible sol-gel composition specifically comprises (i) 0.5 to 15.0% by mass of a gelatin having a Bloom number of 60 to 325, (ii) a nanoparticulate system comprising at least one selected from the group consisting of a biological polymer, a synthetic polymer, a lipid, a metal core, and a silica core (iii) a surfactant, and (iv) a biocompatible solvent, wherein the mass ratio [gelatin]/[surfactant(s)] is 0.5 to 10.0, said thermoreversible sol-gel composition exhibits a gel formation temperature as well as a gel melting temperature in the above-defined specific ranges.

The thermoreversible sol-gel composition of the present invention comprises 0.5 to 15.0% by mass, preferably 1.0 to 9.5% by mass, more preferably 1.2 to 5.0% by mass, and most preferably 1.4 to 3.5% by mass, of a gelatin having a Bloom number of 60 to 325. For example, the thermoreversible sol-gel composition of the present invention comprises 0.5 to 9.5% by mass, 0.5 to 5.0% by mass, 0.5 to 3.5% by mass, 1.0 to 15.0% by mass, 1.0 to 5.0% by mass, 1.0 to 3.5% by mass, 1.2 to 15.0% by mass, 1.2 to 9.5% by mass, 1.2 to 3.5% by mass, 1.4 to 15.0% by mass, 1.4 to 9.5% by mass, or 1.4 to 5.0% by mass of a gelatin having a Bloom number of 60 to 325.

The gelatin according to the present invention is not further limited, provided that said gelatin has a Bloom number of 60 to 325. Generally, a gelatin represents a biopolymer material composed of polypeptide chains of varying length. Preferably, the gelatin of the present invention is at least one selected from the group consisting of type A gelatins and type B gelatins. Within the gelatin industry, gelatin is produced by the following general production steps (i) pretreatment, (ii) hydrolysis, (iii) extraction, and (iv) recovery, which is commonly known by a person skilled in the art. In this context, during said hydrolysis step, the gelatin obtained from an acid-treated raw material is called type-A gelatin and the gelatin obtained from an alkali-treated raw material is referred to as type-B gelatin.

The Bloom number of a gelatin can be measured by the Bloom test, which is commonly known by a person skilled in the art. Said Bloom test determines the weight in grams needed by a specified plunger (normally with a diameter of 1.27 cm) to depress the surface of the (gelatin) gel by 4 mm without breaking it at a specified temperature. The number of grams is called the Bloom number. Specifically, to perform the Bloom test on gelatin, an 6.67% aqueous gelatin solution is kept for 17 to 18 hours at 10.0°C prior to being tested.

According to the present invention, the Bloom number of the gelatin is 60 to 325, preferably 220 to 320, more preferably 240 to 310, most preferably 260 to 300. For instance, the Bloom number of the gelatin according to the present invention may be 60 to 320, 60 to 310, 60 to 300, 220 to 325, 220 to 310, 220 to 300, 240 to 325, 240 to 320, 240 to 300, 260 to 325, 260 to 320, or 260 to 310.

In a preferred embodiment of the present invention, the gelatin has a number average molecular weight (Mₙ) of 20000 to 100000 g/mol, more preferably 40000 to 90000 g/mol, and most preferably 60000 to 80000 g/mol. For instance, the number average molecular weight (Mₙ) of the gelatin is 20000 to 90000 g/mol, 20000 to 80000 g/mol, 40000 to 100000 g/mol, 40000 to 80000 g/mol, 60000 to 100000 g/mol, or 60000 to 90000 g/mol.

The number average molecular weight (Mₙ) of the gelatin can be determined by any method known by a person skilled in the art. Specifically, the Mₙ of the gelatin can be determined by by size exclusion chromatography (SEC).

Moreover, according to the present invention, the thermoreversible sol-gel composition comprises a nanoparticulate system comprising at least one selected from the group consisting of a biological polymer, a synthetic polymer, a lipid, a metal core, and a silica core. Generally, the term nanoparticulate systems refers to all kinds of particles and fibers having an average particle size/diameter of the cross-section of the fibers of 1 to 500 nm. According to the present invention, the nanoparticulate system is not further limited if it comprises at least one selected from the group consisting of a biological polymer, a synthetic polymer, a lipid, a metal core, and a silica core. Moreover, the nanoparticulate system can be one type of a nanoparticulate system or two or more types of nanoparticulate systems. Most preferably, the nanoparticulate system of the present invention comprises a biological polymer and/or a synthetic polymer.

For instance, said nanoparticulate system can be polymer nanoparticles (matrix, core-shell, micelles), liposomes, dendrimers, solid lipid nanoparticles, mesoporous silica nanoparticles, mesoporous magnetic colloidal nanocrystal clusters, metal-organic frameworks, polymer-drug conjugates, metal-biomolecule frameworks, metal nanoparticles (e.g. Ag, Au), metal-oxide-nanoparticles (e.g. Cu/CuO, CO/CO₃O₄, ZnO, Mn₂O₃, MgO), and/or other nano-scale systems.

According to the present invention, (i) a nanoparticulate system comprising a biological polymer and/or a synthetic polymer can be polymer-drug conjugates, dendrimers, and/or polymer nanoparticles (matrix, core-shell type, micelles), (ii) a nanoparticulate system comprising a lipid can be liposomes and/or solid lipid nanoparticles, (iii) a nanoparticulate system comprising a metal core can be metal-biomolecule frameworks, mesoporous magnetic colloidal nanocrystal clusters, metal nanoparticles (e.g. Ag, Au), and/or metal-oxide-nanoparticles (e.g. Cu/CuO, CO/Co₃O₄, ZnO, Mn₂O₃, MgO), and (iv) a nanoparticulate system comprising a silica core can be mesoporous silica nanoparticles.

Preferably, the biological polymer and/or synthetic polymer is at least one selected from the group consisting of a polymethacrylate, a polyalkylacrylate, a polyether, a polyester, a polyketone, a polyolefin, a polyurethane, a polyamide, a polyamine, a polyurea, a polysiloxane, a polyvinylidene, an oligosaccharide, a polysaccharide, an oligopeptide, a polypeptide, an oligonucleotide, derivatives thereof, and copolymers thereof. For instance, said biological polymer and/or synthetic polymer may be poly (lactic acid), poly (lactic-co-glycolic acid), poly (*ε*-caprolactone), chitosan, and poly (alkyl cyanoacrylates). However, the given invention is not limited to a specific type of polymer.

According to a preferred embodiment of the present invention, the biological polymer is at least one selected from the group consisting of an oligosaccharide, a polysaccharide, an oligopeptide, a polypeptide, an oligonucleotide, derivatives thereof, and copolymers thereof. Preferably, the synthetic polymer is at least one selected from the group consisting of a poly (meth)acrylate, a polyalkylacrylate, a polyether, a polyester, a polyketone, a polyolefin, a polyurethane, a polyamide, a polyamine, a polyurea, a polysiloxane, a polyvinylidene.

Said biological polymer and/or synthetic polymer according to the present invention preferably has a number average molecular weight (Mₙ) of 500 to 500000 g/mol, more preferably 1000 to 300000 g/mol, still more preferably 1500 to 100000 g/mol, most preferably 2000 to 50000 g/mol. The biological polymer and/or synthetic polymer may has a number average molecular weight (Mₙ) of 500 to 300000 g/mol, 500 to 100000 g/mol, 500 to 50000 g/mol, 1000 to 500000 g/mol, 1000 to 100000 g/mol, 1000 to 50000 g/mol, 1500 to 500000 g/mol, 1500 to 300000 g/mol, 1500 to 50000 g/mol, 2000 to 500000 g/mol, 2000 to 300000 g/mol, or 2000 to 100000 g/mol.

The number average molecular weight (Mₙ) of the biological polymer and/or synthetic polymer can be determined by any method known by a person skilled in the art. Specifically, the Mₙ of the biological and/or synthetic polymer can be determined by static light scattering (SLS) and/or size exclusion chromatography (SEC).

According to the present invention, in view of reducing agglomeration of the nanoparticulate system in the thermoreversible sol-gel composition in sol-form, the thermoreversible sol-gel composition preferably comprises a nanoparticulate system having an average particle size of 1 to 500 nm, more preferably 10 to 400 nm, still more preferably 30 to 300, most preferably 40 to 200 nm. For example, the nanoparticulate system exhibit an average particle size of 1 to 400 nm, 1 to 300 nm, 1 to 200, 10 to 500 nm, 10 to 300 nm, 10 to 200 nm, 30 to 500 nm, 30 to 400 nm, 30 to 200 nm, 40 to 500 nm, 40 to 400 nm, or 40 to 300 nm.

Furthermore, according to the present invention, the shape and/or morphology of said nanoparticulate system is not particularly limited, according to which they can represent, for example, spherical particles, cubic particles, or fibers. If the nanoparticulate system according to the present invention are present in form of fibers, the average particle size corresponds to the average diameter of the cross-section of these fibers.

Moreover, in view of reducing agglomeration of the nanoparticulate system in the thermoreversible sol-gel composition in sol-form, the thermoreversible sol-gel composition preferably comprises a nanoparticulate system having a polydispersity index of less than 0.25, more preferably less than 0.20, still more preferably less than 0.15, most preferably less than 0.12.

Cryogenic transmission electron microscopy (Cryo-TEM) can be used to characterize the average particle size, the morphology/shape, and the polydispersity index of the nanoparticulate system. Therefore, 4 µl of the respective sample to be analyzed was placed on a carbon-coated support grid. After 3 seconds, an excess of the sample solution was removed with a filter paper (Whatman, Germany). Subsequently, the carbon-coated support grid carrying the sample was frozen in liquid ethane at -180°C. The targeted images were acquired using a CM200FEG microscope equipped with a high-resolution camera (4Kx 4K TemCam F416 CMOS). For all image acquisitions, the accelerating voltage was 200 kV and a low-dose system (20-30 e⁻/Å²) was used. Detailed images were taken at 50000 times magnification and overview images at 5000 times magnification.

Moreover, the average particle size and the polydispersity index of the nanoparticulate system can be determined by dynamic light scattering (DLS) using a Zetasizer Nano ZS (Malvern Panalytical Ltd, Malvern,UK).

According to the present invention the thermoreversible sol-gel composition preferably comprises 0.1 to 5.0% by mass, more preferably 0.5 to 4.5% by mass, still more preferably 1.0 to 4.0% by mass, most preferably 1.5 to 3.5% by mass of the nanoparticulate system. For instance, the thermoreversible sol-gel composition may comprise 0.1 to 4.5% by mass, 0.1 to 4.0% by mass, 0.1 to 3.5% by mass, 0.5 to 5.0% by mass, 0.5 to 4.0% by mass, 0.5 to 3.5% by mass, 1.0 to 5.0% by mass, 1.0 to 4.5% by mass, 1.0 to 3.5% by mass, 1.5 to 5.0% by mass, 1.5 to 4.5% by mass, or 1.5 to 4.0% by mass of the nanoparticulate system.

In addition, the thermoreversible sol-gel composition preferably comprises a nanoparticulate system comprising at least one selected from the group consisting of the above-described biological polymer, synthetic polymer, lipid, a metal core, and a silica core, as well as further comprises an active pharmaceutical ingredient. According to the present invention, said active pharmaceutical ingredient is not further limited if it is suitable for being incorporated by nanoparticulate systems and use in medicine. Said active pharmaceutical ingredient can be one type of an active pharmaceutical ingredient or two or more types of pharmaceutical ingredients.

The active pharmaceutical ingredient may be one or more selected from the group consisting of human growth hormone, growth hormone releasing hormone, growth hormone releasing peptide, interferons, colony stimulating factors, interleukins, macrophage activating factor, macrophage peptide, B cell factor, T cell factor, protein A, allergy inhibitor, cell necrosis glycoproteins, immunotoxin, lymphotoxin, tumor necrosis factor, tumor suppressors, metastasis growth factor, alpha-1 antitrypsin, albumin and fragment polypeptides thereof, apolipoprotein-E, erythropoietin, factor VII, factor VIII, factor IX, plasminogen activating factor, urokinase, streptokinase, protein C, C-reactive protein, renin inhibitor, collagenase inhibitor, superoxide dismutase, platelet-derived growth factor, epidermal growth factor, osteogenic growth factor, bone stimulating protein, calcitonin, insulin, atriopeptin, cartilage inducing factor, connective tissue activating factor, follicle stimulating hormone, luteinizing hormone, luteinizing hormone releasing hormone, nerve growth factors, parathyroid hormone, relaxin, secretin, somatomedin, insulin-like growth factor, adrenocortical hormone, glucagon, cholecystokinin, pancreatic polypeptide, gastrin releasing peptide, corticotropin releasing factor, thyroid stimulating hormone, monoclonal or polyclonal antibodies against various viruses, bacteria, or toxins, virus-derived vaccine antigens, octreotide, exenatid, teriparatide, vancomycin, cyclosporine, rifampycin, lopinavir, ritonavir, vancomycin, telavancin, oritavancin, dalbavancin, bisphosphonates, itraconazole, danazol, paclitaxel, cyclosporin, naproxen, capsaicin, albuterol sulfate, terbutaline sulfate, diphenhydramine hydrochloride, chlorpheniramine maleate, loratidine hydrochloride, fexofenadine hydrochloride, phenylbutazone, nifedipine, carbamazepine, naproxen, cyclosporin, betamethosone, danazol, dexamethasone, prednisone, hydrocortisone, 17 beta-estradiol, ketoconazole, mefenamic acid, beclomethasone, alprazolam, midazolam, miconazole, ibuprofen, ketoprofen, prednisolone, methylprednisone, phenytoin, testosterone, flunisolide, diflunisal, budesonide, fluticasone, insulin, glucagon-like peptide, C-Peptide, erythropoietin, calcitonin, lutenizing hormone, prolactin, adrenocorticotropic hormone, leuprolide, interferon alpha-2b, interferon beta-la, sargramostim, aldesleukin, interferon alpha-2a, interferon alpha-n3alpha-proteinase inhibitor, etidronate, nafarelin, chorionic gonadotropin, prostaglandin E2, epoprostenol, acarbose, metformin, desmopressin, cyclodextrin, antibiotics, antifungal drugs, steroids, anticancer drugs, analgesics, anti-inflammatory agents, anthelmintics, anti-arrhythmic agents, penicillins, anticoagulants, antidepressants, antidiabetic agents, antiepileptics, antihistamines, antihypertensive agents, antimuscarinic agents, antimycobacterial agents, antineoplastic agents, immunosuppressants, antithyroid agents, antiviral agents, anxiolytic sedatives, hypnotics, neuroleptics, astringents, beta-adrenoceptor blocking agents, blood products and substitutes, cardiacinotropic agents, contrast media, corticosteroids, cough suppressants, expectorants, mucolytics, diuretics, CNS-active compounds, dopaminergics, antiparkinsonian agents, haemostatics, immunological agents, lipid regulating agents, muscle relaxants, parasympathomimetics, parathyroid calcitonin, prostaglandins, radiopharmaceuticals, sex hormones, steroids, anti-allergic agents, stimulants, anoretics, sympathomimetics, thyroid agents, vasidilators, xanthines, heparins, therapeutic oligonucleotides, somatostatins and analogues thereof, and pharmacologically acceptable organic and inorganic salts or metal complexes thereof.

According to the present invention, the nanoparticulate system preferably comprise 0.1 to 80.0% by mass, more preferably 2.0 to 70.0% by mass, still more preferably 4.0 to 60.0% by mass, most preferably 6.0 to 50.0% by mass of the active pharmaceutical ingredient. Specifically, the nanoparticulate system of the present invention may comprise 0.1 to 70.0% by mass, 0.1 to 60.0% by mass, 0.1 to 50.0% by mass, 2.0 to 80.0% by mass, 2.0 to 60.0% by mass, 2.0 to 50.0% by mass, 4.0 to 80.0% by mass, 4.0 to 70.0% by mass, 4.0 to 50.0% by mass, 6.0 to 80.0% by mass, 6.0 to 70.0% by mass, or 6.0 to 60.0% by mass of the active pharmaceutical ingredient.

According to the present invention, the thermoreversible sol-gel composition comprises a biocompatible solvent essentially comprising water. In this context, "essentially comprising water" means the biocompatible solvent comprises 90% by mass or more, preferably 93% by mass or more, more preferably 95% by mass or more, still more preferably 97% by mass or more, and most preferably 99% by mass or more of water, wherein water may be demineralized water or tap water. Preferably, the biocompatible solvent further comprises at least one selected from the group consisting of ethanol, sodium chloride or other salts, buffer compounds, polyethylenglycols and preservants. Specific examples for these preservants are thimerosal, propyl-4-hydroxy-benzoat, chlorocresol, benzylalcohol, chlorobutanol, phenoxyethanol and propylparaben.

Preferably, the thermoreversible sol-gel composition of the present invention comprises 78 to 97% by mass, more preferably 89 to 96% by mass, still more preferably 90 to 95% by mass, most preferably 91 to 94% by mass of the biocompatible solvent, which essentially comprises water. For instance, the thermoreversible sol-gel composition comprises 88 to 96% by mass, 88 to 95% by mass, 88 to 94% by mass, 89 to 97% by mass, 89 to 95% by mass, 89 to 94% by mass, 90 to 97% by mass, 90 to 96% by mass, 90 to 94% by mass, 91 to 97% by mass, 91 to 96% by mass, or 91 to 95% by mass of the biocompatible solvent, which essentially comprises water.

According to the present invention, the thermoreversible sol-gel composition comprises a surfactant. The surfactant of the present invention is not further limited if said surfactant is suitable to support the formulation of the above-described nanoparticulate systems. Said surfactant can be one type of a surfactant or two or more types of surfactants. The surfactant of the present invention is preferably at least one selected from the group consisting of an anionic surfactant, a cationic surfactant, a non-ionic surfactant, an amphoteric surfactant, and mixtures thereof. Specific examples of the surfactant of the present invention are sodium dodecyl sulfate (SDS), sodium cholate, sodium deoxycholate, potassium oleate, oleic acid, stearic acid and docusate sodium.

Preferably, the thermoreversible sol-gel composition comprises 0.2 to 5.0% by mass, more preferably 0.3 to 4.5% by mass, still more preferably 0.4 to 3.5% by mass, most preferably 0.5 to 3.0% by mass of the above-described surfactant. For instance, the thermoreversible sol-gel composition comprises 0.2 to 4.5% by mass, 0.2 to 3.5% by mass, 0.2 to 3.0% mass, 0.3 to 5.0% by mass, 0.3 to 3.5% by mass, 0.3 to 3.0% by mass, 0.4 to 5.0% by mass, 0.4 to 4.5% by mass, 0.4 to 3.0% by mass, 0.5 to 5.0% by mass, 0.5 to 4.5% by mass, or 0.5 to 3.5% by mass of the above-described surfactant.

Moreover, according to the present invention, in the thermoreversible sol-gel composition the mass ratio [gelatin]/[surfactant(s)] is 0.5 to 10.0, preferably 1.0 to 8.0, more preferably 1.5 to 6.0, still more preferably 1.7 to 5.0, most preferably 2.0 to 4.0. In this context the term "[gelatin]" represents the % by mass of gelatin comprised by the thermoreversible sol-gel composition and the expression "[surfactant(s)]" represents the % by mass of surfactant(s) comprised by the thermoreversible sol-gel composition. Exemplary, in the thermoreversible sol-gel composition, the mass ratio [gelatin]/[surfactant(s)] is 0.5 to 8.0, 0.5 to 6.0, 0.5 to 5.0, 0.5 to 4.0, 1.0 to 10.0, 1.0 to 6.0, 1.0 to 5.0, 1.0 to 4.0, 1.5 to 10.0, 1.5 to 8.0, 1.5 to 5.0, 1.5 to 4.0, 1.7 to 10.0, 1.7 to 8.0, 1.7 to 6.0, 1.7 to 4.0, 2.0 to 10.0, 2.0 to 8.0, 2.0 to 6.0, or 2.0 to 5.0.

In another aspect, the present invention provides a pharmaceutical composition, comprising the above-described thermoreversible sol-gel composition for stability enhancement and ready-to-use liquid application of nanoparticulate systems, wherein the nanoparticulate system further comprises the above-defined active pharmaceutical ingredient. According to the present invention, the pharmaceutical composition is not further limited if it comprises the themoreversible sol-gel composition of the present invention, wherein the nanoparticulate system further comprises the above-described active pharmaceutical ingredient. In addition, the pharmaceutical composition may further comprise at least one selected from the group consisting of sodium chloride or other salts, buffer compounds, polyethylenglycols and the above-defined preservants.

The above-defined thermoreversible sol-gel composition of the present invention can be produced by a method, comprising in that order: (i) providing a nanoparticulate system suspension comprising a biocompatible solvent essentially comprising water, a surfactant, and a nanoparticulate system comprising at least one selected from the group consisting of a biological polymer, a synthetic polymer, a lipid, a metal core, and a silica core; (ii) adding 0.5 to 15.0% by mass of a gelatin having a Bloom number of 60 to 325 to said nanoparticulate system suspension, wherein the mass ratio [gelatin]/[surfactant(s)] is 0.5 to 10.0; (iii) homogenizing the mixture comprising the nanoparticulate system, the surfactant, the gelatin, and the biocompatible solvent to form a gelatin-nanoparticulate system formulation; and (iv) cooling the gelatin-nanoparticulate system formulation to a temperature of 2.0 to 10.0°C.

The above-defined method of producing the thermoreversible sol-gel composition of the present invention comprises a step of (i) providing a nanoparticulate system suspension comprising a biocompatible solvent essentially comprising water, a surfactant, and a nanoparticulate system comprising at least one selected from the group consisting of a biological polymer, a synthetic polymer, a lipid, a metal core, and a silica core. Said providing step of a nanoparticulate system suspension is not particularly limited provided that said nanoparticulate system suspension comprises the above-defined biocompatible solvent essentially comprising water, the above-described surfactant, and the above-characterized nanoparticulate system comprising at least one selected from the group consisting of a biological polymer, a synthetic polymer, a lipid, a metal core, and a silica core. In this context, methods of producing nanoparticulate system suspensions are commonly known by a person skilled in the art.

The nanoparticulate system suspension may be provided by a method of producing a nanoparticulate system suspension, comprising: (a) providing a polymer solution comprising the above-defined biological polymer and/or the above-defined synthetic polymer and optionally the above-described active pharmaceutical ingredient dissolved in the below-characterized organic solvent; (b) providing a surfactant solution comprising the above-characterized biocompatible solvent essentially comprising water and the above-described surfactant; (c) mixing said polymer solution with said surfactant solution by ultra-sonification to form a surfactant-polymer emulsion; (d) after said mixing, removing the organic solvent by evaporation to form a gelatin-nanoparticulate system formulation; and optionally (e) after said evaporation, removing the non-encapsulated active pharmaceutical ingredient by filtration.

In addition, the above-defined method of producing the thermoreversible sol-gel composition of the present invention comprises a step of (ii) adding 0.5 to 15.0% by mass of a gelatin having a Bloom number of 60 to 325 to said nanoparticulate system suspension, wherein the mass ratio [gelatin]/[surfactant(s)] is 0.5 to 10.0. In this context, the gelatin and the mass ratio [gelatin]/[surfactant(s)] are equal to the above-defined gelatin and mass ratio [gelatin]/[surfactant(s)] of the thermoreversible sol-gel composition of the present invention. Said step of adding is not particularly limited if the gelatin is added to the nanoparticulate system suspension. The gelatin may be added to the nanoparticulate system suspension as a gelatin or as a gelatin solution, wherein the gelatin solution comprises the gelatin dissolved in the above-described biocompatible solvent essentially comprising water. Preferably, after said adding step, the gelatin can be allowed to swell for 1 to 60 min, more preferably 5 to 50 min, still more preferably 10 to 40 min, most preferably 15 to 30 min.

Moreover, the above-defined method of producing the thermoreversible sol-gel composition of the present invention comprises a step of (iii) homogenizing the mixture comprising the nanoparticulate system, the surfactant, the gelatin, and the biocompatible solvent to form a gelatin-nanoparticulate system formulation. In this context, the gelatin-nanoparticulate system formulation is not further limited, provided that it comprises the above-described nanoparticulate system comprising at least one selected from the group consisting of a biological polymer, a synthetic polymer, a lipid, a metal core, and a silica core, optionally the above-defined active pharmaceutical ingredient, the above-defined surfactant essentially surrounding said nanoparticulate system for stabilization of said nanoparticulate system, 0.5 to 15.0% by mass of the above-defined gelatin having a Bloom number of 60 to 325, and the above outlined biocompatible solvent essentially comprising water, wherein the mass ratio [gelatin]/[surfactant(s)] is 0.5 to 10.0.

The homogenizing of the above-described mixture is not further limited provided that the mixture is homogenized. For instance, said homogenizing step may be performed using a heatable shaker, for example a Thermomixer^{®} (R 5355 Eppendorf, Germany) or a speed mixer, for example a SpeedMixer^{®} (Hauschild GmbH & Co. KG, Germany), depending on the specific type of nanoparticulate system. In case of shear-sensitive nanoparticulate systems, homogenizing of the mixture using a heatable shaker is preferred, and in case of heat-sensitive nanoparticulate systems, homogenizing of the mixture using a speed mixer is preferred.

Moreover, the above-defined method of producing the thermoreversible sol-gel composition of the present invention comprises a step of (iv) cooling the gelatin-nanoparticulate system formulation to a temperature of 2.0 to 10.0°C. Said cooling step is not particularly limited and is equal to the cooling of the below-described in-situ method of producing the thermoreversible sol-gel composition of the present invention.

Besides, the above-described method of producing the thermoreversible sol-gel composition of the present invention may further comprise (v) after said cooling, holding the gelatin-nanoparticulate system formulation at said temperature of 2.0 to 10.0°C for 1.5 to 5.0 hours, preferably 1.7 to 4.5 hours, more preferably 2.0 to 4.0 hours, most preferably 2.2 to 3.5 hours. For instance, after said cooling, the gelatin-nanoparticulate system formulation may be hold at said temperature of 2.0 to 10.0°C for 1.5 to 4.5 hours, 1.5 to 4.0 hours, 1.5 to 3.5 hours, 1.7 to 5.0 hours, 1.7 to 4.0 hours, 1.7 to 3.5 hours, 2.0 to 5.0 hours, 2.0 to 4.5 hours, 2.0 to 3.5 hours, 2.2 to 5.0 hours, 2.2 to 4.5 hours, or 2.2 to 4.0 hours. Upon completion of the gel formation the resulting formulation/system can be stored at said temperature of 2.0 to 10.0°C till use/application.

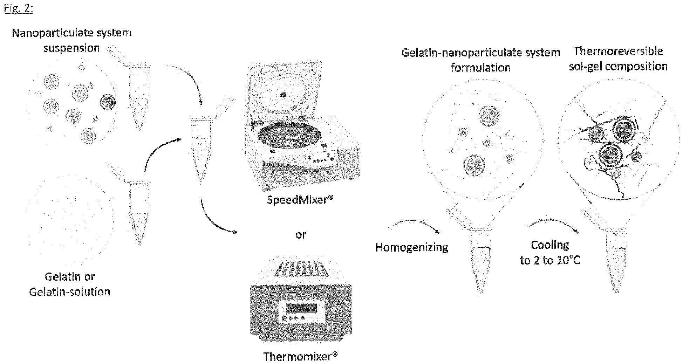

The present invention provides further an in-situ method of producing the thermoreversible sol-gel composition of the present invention, wherein the nanoparticulate system comprises a biological polymer and/or a synthetic polymer, comprising: (i) providing a polymer solution comprising a biological polymer and/or a synthetic polymer dissolved in an organic solvent; (ii) providing a gelatin-surfactant solution comprising a biocompatible solvent essentially comprising water, 0.5 to 15.0% by mass of a gelatin having a Bloom number of 60 to 325, and a surfactant, wherein the mass ratio [gelatin]/[surfactant(s)] is 0.5 to 10.0; (iii) mixing the polymer solution with the gelatin-surfactant solution by ultra-sonification to form a gelatin-surfactant-polymer emulsion; (iv) after said mixing, removing the organic solvent by evaporation to form a gelatin-nanoparticulate system formulation; and (v) after said evaporation, cooling the gelatin-nanoparticulate system formulation to a temperature of 2.0 to 10.0°C. In this context, an exemplary sequence of the above-defined in-situ method of producing the thermoreversible sol-gel composition of the present invention is illustrated in Figure 2.

The in-situ method of producing the thermoreversible sol-gel composition of the present invention comprises (i) providing a polymer solution comprising a biological polymer and/or a synthetic polymer dissolved in an organic solvent. According to the present invention, said polymer solution is not particularly limited if it comprises a biological polymer and/or a synthetic polymer dissolved in an organic solvent. In this context, the biological polymer and/or the synthetic polymer is equal to the above-defined biological polymer and/or synthetic polymer of the thermoreversible sol-gel composition of the present invention. According to the present invention, said polymer solution can be provided by dissolving the biological polymer and/or the synthetic polymer in the organic solvent during mixing of said biological polymer and/or said synthetic polymer with said organic solvent.

Furthermore, according to the present invention, the organic solvent is not particularly limited provided that it is able to dissolve said biological polymer and/or said synthetic polymer. According to the present invention, said organic solvent can be one kind of solvent or two or more kinds of solvents. Preferably, the organic solvent is at least one selected from the group consisting of an alcohol, an ester, an ether, a ketone, a substituted or unsubstituted linear hydrocarbon, a substituted or unsubstituted cyclic hydrocarbon, and a mixture thereof. For instance, the organic solvent in the providing step of the polymer solution may be diethyl ether, chloroform, or dichloromethane.

According to the present invention, the polymer solution preferably further comprises an active pharmaceutical ingredient dissolved in said organic solvent. In this context, the active pharmaceutical ingredient is not particularly limited, provided that it can be dissolved in the organic solvent and is equal to the above-defined active pharmaceutical ingredient of the thermoreversible sol-gel composition of the present invention.

Besides, the in-situ method of producing the thermoreversible sol-gel composition of the present invention comprises (ii) providing a gelatin-surfactant solution comprising a biocompatible solvent essentially comprising water, 0.5 to 15.0% by mass of a gelatin having a Bloom number of 60 to 325, and a surfactant, wherein the mass ratio [gelatin]/[surfactant(s)] is 0.5 to 10.0.

According to the present invention, said gelatin-surfactant solution is not particularly limited if it comprises a biocompatible solvent essentially comprising water, 0.5 to 15.0% by mass of a gelatin having a Bloom number of 60 to 325, and a surfactant, wherein the mass ratio [gelatin]/[surfactant(s)] is 0.5 to 10.0. In this context, the biocompatible solvent, the gelatin, the surfactant, and the mass ratio [gelatin]/[surfactant(s)] are equal to the above-defined biocompatible solvent, gelatin, surfactant, and mass ratio [gelatin]/[surfactant(s)] of the thermoreversible sol-gel composition of the present invention, respectively. According to the present invention, said gelatin-surfactant solution can be provided by dissolving 0.5 to 15.0% by mass of the gelatin having a Bloom number of 60 to 325 and the surfactant in the biocompatible solvent essentially comprising water during mixing of said gelatin and said surfactant with said biocompatible solvent, provided that the mass ratio [gelatin]/[surfactant(s)] is 0.5 to 10.0.

Furthermore, the in-situ method of producing the thermoreversible sol-gel composition of the present invention comprises (iii) mixing the polymer solution with the gelatin-surfactant solution by ultra-sonification to form a gelatin-surfactant-polymer emulsion. According to the present invention, the gelatin-surfactant-polymer emulsion is not particularly limited if it is an emulsion comprising the above-defined polymer solution and gelatin-surfactant solution. Moreover, according to the present invention, ultra-sonification is not further limited and common ultra-sonification techniques are known to a person skilled in the art. Ultra-sonification may be provided using an ultrasonic rod (HTU SONI 130, G. Heinemann Ultraschall- und Labortechnik, Schwäbisch Gmünd, Germany).

Preferably, in view of reducing the evaporation of the organic solvent during mixing of the polymer solution with the gelatin-surfactant solution the ultra-sonification is applied for 1 to 10 min, more preferably 2 to 9 min, still more preferably 3 to 8 min, most preferably 4 to 7 min. For instance, during mixing of the polymer solution with the gelatin-surfactant solution the ultra-sonification may be applied for 1 to 9 min, 1 to 8 min, 1 to 7 min, 2 to 10 min, 2 to 8 min, 2 to 7 min, 3 to 10 min, 3 to 9 min, 3 to 7 min, 4 to 10 min, 4 to 9 min, or 4 to 8 min. Preferably, during mixing of the polymer solution with the gelatin-surfactant solution the ultra-sonification is applied with a frequency range of 10 to 80 MHz, more preferably 10 to 40 MHz, most preferably 20 to 40 MHz.

According to the present invention, during mixing of the polymer solution with the gelatin-surfactant solution the ultra-sonification is preferably applied at a temperature of 0.0 to 30.0°C, more preferably 2.5 to 27.5°C, still more preferably 5.0 to 25.0°C, most preferably 7.5 to 22.5°C. For instance, during mixing of the polymer solution with the gelatin-surfactant solution the ultra-sonification can be applied at a temperature of 0.0 to 27.5°C, 0.0 to 25.0°C, 0.0 to 22.5°C, 2.5 to 30.0°C, 2.5 to 25.0°C, 2.5 to 22.5°C, 5.0 to 30.0°C, 5.0 to 27.5°C, 5.0 to 22.5°C, 7.5 to 30.0°C or 7.5 to 27.5°C, or 7.5 to 25.0°C.

In addition, the in-situ method of producing the thermoreversible sol-gel composition of the present invention comprises (iv) after the above-described mixing, removing the organic solvent by evaporation to form a gelatin-nanoparticulate system formulation. According to the present invention, the gelatin-nanoparticulate system formulation is not further limited, provided that it comprises the above-described nanoparticulate system comprising a biological polymer and/or a synthetic polymer and optionally an active pharmaceutical ingredient, the above-defined surfactant essentially surrounding the nanoparticulate system for stabilization, and 0.5 to 15.0% by mass of the above-defined gelatin having a Bloom number of 60 to 325 (partially) swelled with the above outlined biocompatible solvent essentially comprising water, wherein the mass ratio [gelatin]/[surfactant(s)] is 0.5 to 10.0.

According to the present invention, the removing of the organic solvent is not particularly limited if said organic solvent is being evaporated. The evaporation of the organic solvent is preferably performed in a temperature range of 40.0°C lower than the boiling point of the organic solvent to the boiling point of the organic solvent, more preferably 35.0°C lower than the boiling point of the organic solvent to the boiling point of the organic solvent, still more preferably 30.0°C lower than the boiling point of the organic solvent to the boiling point of the organic solvent, most preferably 25.0°C lower than the boiling point of the organic solvent to the boiling point of the organic solvent.

According to the present invention, the evaporation of the organic solvent is preferably be performed for 0.5 to 7.0 hours, more preferably 1.0 to 6.0 hours, still more preferably 1.5 to 5.0 hours, most preferably 2.0 to 4.0 hours in order to ensure the complete evaporation of the organic solvent. Specifically, the evaporation of the organic solvent may be performed for 0.5 to 6.0 hours, 0.5 to 5.0 hours, 0.5 to 4.0 hours, 1.0 to 7.0 hours, 1.0 to 5.0 hours, 1.0 to 4.0 hours, 1.5 to 7.0 hours, 1.5 to 6.0 hours, 1.5 to 4.0 hours, 2.0 to 7.0 hours, 2.0 to 6.0 hours, or 2.0 to 5.0 hours.

Moreover, the in-situ method of producing the thermoreversible sol-gel composition of the present invention comprises (v) after said evaporation, cooling the gelatin-nanoparticulate system formulation to a temperature of 2.0 to 10.0°C. According to the present invention, said cooling step is not particularly limited provided that the gelatin-nanoparticulate system formulation is cooled to 2.0 to 10.0°C. The cooling of the gelatin-nanoparticulate system formulation may be performed using a refrigerator.

According to the present invention, the in-situ method of producing the thermoreversible sol-gel composition of the present invention may further comprise (vi) after said cooling, holding the gelatin-nanoparticulate system formulation at said temperature of 2.0 to 10.0°C for 1.5 to 5.0 hours, preferably 1.7 to 4.5 hours, more preferably 2.0 to 4.0 hours, most preferably 2.2 to 3.5 hours. For instance, after said cooling, the gelatin-nanoparticulate system formulation may be hold at said temperature of 2.0 to 10.0°C for 1.5 to 4.5 hours, 1.5 to 4.0 hours, 1.5 to 3.5 hours, 1.7 to 5.0 hours, 1.7 to 4.0 hours, 1.7 to 3.5 hours, 2.0 to 5.0 hours, 2.0 to 4.5 hours, 2.0 to 3.5 hours, 2.2 to 5.0 hours, 2.2 to 4.5 hours, or 2.2 to 4.0 hours. Upon completion of the gel formation the resulting formulation/system can be stored at said temperature of 2.0 to 10.0°C till use/application.

In the above-defined in-situ method of producing the thermoreversible sol-gel composition of the present invention, the nanoparticulate system can be prepared advantageously *in situ* with the swelling of the gelatin in the biocompatible solvent essentially comprising water, since, for instance, the above-described mixing step of (iii) mixing the polymer solution with the gelatin-surfactant solution by ultra-sonification to form a gelatin-surfactant-polymer emulsion does not affect or change the specific properties of the gelatin. Thus, the above-described in-situ method of producing the thermoreversible sol-gel composition of the present invention advantageously is cost-saving, user-friendly, as well as timesaving.

In another aspect of the present invention, the present invention relates to the above-defined thermoreversible sol-gel composition of the present invention for use in medicine, wherein the nanoparticulate system further comprises the above-described active pharmaceutical ingredient.

For use in medicine, the thermoreversible sol-gel composition of the present invention, wherein the nanoparticulate system further comprises the above-described active pharmaceutical ingredient, can be administered rectal, oral, or parenteral to a patient as a liquid ready-to-use composition at standard room temperature conditions without additional prior preparation steps by medical staff, e.g. redispersing the lyophilizate prior to use.

In a preferred embodiment of the present invention, the above-defined thermoreversibe sol-gel composition of the present invention is topically applied. Herein, a topical application represents a medication that is applied to a particular place on or in the body of a patient, for instance, to mucous membranes or skin.

In still another aspect of the present invention, the present invention relates to the thermoreversible sol-gel composition of the present invention for use in treating *ulcerative colitis,* wherein the nanoparticulate system further comprises budesonide (BUD) in form of a nanocrystal formulation. In case of treatment of *ulcerative colitis* using the above-described thermoreversible sol-gel composition of the present invention, the distribution and release behaviour of the nanoparticulate systems can be enhanced as well as the wound healing of the respective damaged mucosa can be improved.

According to the present invention, a thermoreversible sol-gel composition for stability enhancement and ready-to-use liquid application of nanoparticulate systems is provided. In this context, stability enhancement of nanoparticulate systems means, for instance, (i) prevent/reduce an increase of the polydispersity index and/or the average particle size of the nanoparticulate system due to agglomeration of said nanoparticulate systems, and/or (ii) prevent/reduce a decrease of drug load of the nanoparticulate system under stress conditions and long-term storage. Moreover, a ready-to-use liquid application refers to, for instance, a rectal, oral, or parenteral administration to a patient at standard room temperature conditions without additional prior preparation steps by medical staff, *i.e.* redispersing the lyophilizate prior to use.

The present invention described herein offers a stable carrier system for colloidal drugs and nanoparticulate systems that enable an improved long-term storage while retaining the benefits of a liquid ready-to-use formulation by embedding the drug carrier system in a newly developed gel matrix, based on gelatin, a biocompatible solvent, and a variety of surfactants. The interaction between gelatin and the surfactant allows a modulation of both, the gel formation temperature and gel melting temperature of the gel matrix. This provides an effective and thermoreversible carrier matrix for any kind of nanoparticulate systems, which can be stored easily as a solid and stable gel at low temperatures, e.g. 2.0 to 10.0 °C, and quickly liquefied again at moderate temperatures, e.g. 15.0 to 25.0 °C, without the need of any technical equipment. This reduces the risk of incorrect application and can help to improve dosing accuracy. Neither the embedding process nor the thermally induced liquefaction affects the integrity of the respective nanoparticulate system. Long-term storage experiments showed significant advantages of the gel-matrix formulation regarding average particle size, polydispersity index, and drug load.

According to the present invention, if said thermoreversible sol-gel composition exhibits a gel formation temperature as well as a gel melting temperature in the above-defined specific ranges, said thermoreversible sol-gel composition can be effectively used as a stable carrier system for colloidal drugs and nanoparticulate systems that enable an improved long-term storage while retaining the benefits of a liquid ready-to-use formulation. In contrast, if the thermoreversible sol-gel composition exhibits a gel formation temperature lower than 2.0°C, the long-term storage and transport costs of the thermoreversible sol-gel composition increase. Moreover, if the thermoreversible sol-gel composition exhibits a gel melting temperature higher than 25.0°C, the thermoreversible sol-gel composition does not represent a liquid ready-to-use formulation, e.g. for administering to a patient, at standard room temperature conditions.

In addition to the embedding of pre-produced nanoparticulate systems in the gel matrix, according to the present invention the gel matrix and the nanoparticulate systems can be produced in a one-step/in-situ process. This new formulation approach offers not only the potential to stabilize a wide range of colloidal drug carriers, but also maintains the liquid character for application. This advantage in combination with a user-friendliness, time saving, and inexpensive production makes the invented carrier system a very promising and efficient alternative to the currently available stabilization methods for nanoparticulate system-based dosage forms.

The figures show:

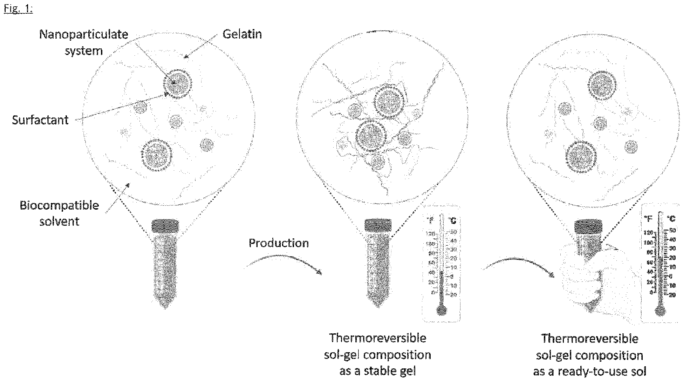

- Fig. 1:: Schematic illustration of the concept of the thermoreversible sol-gel composition of the present invention as a stable carrier system for colloidal drugs and nanoparticulate systems that enable an improved long-term storage while retaining the benefits of a liquid ready-to-use formulation.
- Fig. 2:: Schematic illustration of a method of producing the thermoreversible sol-gel composition.

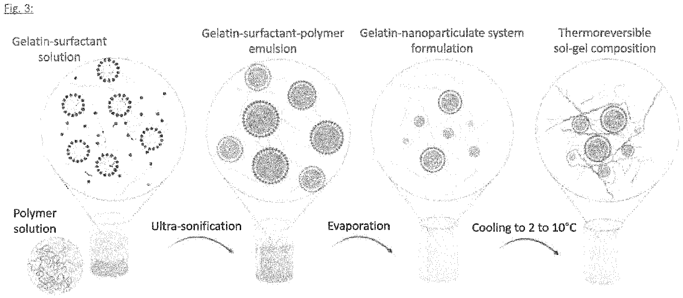

- Fig. 3:: Schematic illustration of the in-situ method of producing the thermoreversible solgel composition of the present invention.

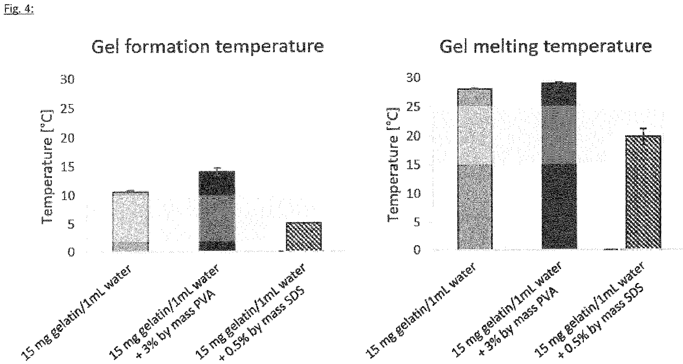

- Fig. 4:: Gel formation temperature (left) and the corresponding gel melting temperature (right) of different gel matrix compositions of a gelatin having a Bloom number of 300 with SDS, PVA, or without additive. In this context, the grey overlays in the graphs indicate the refrigerator temperature from 2.0 to 10.0°C (left) and the room temperature range from 15.0 to 25.0°C (right) as defined by European Pharmacopoeia.

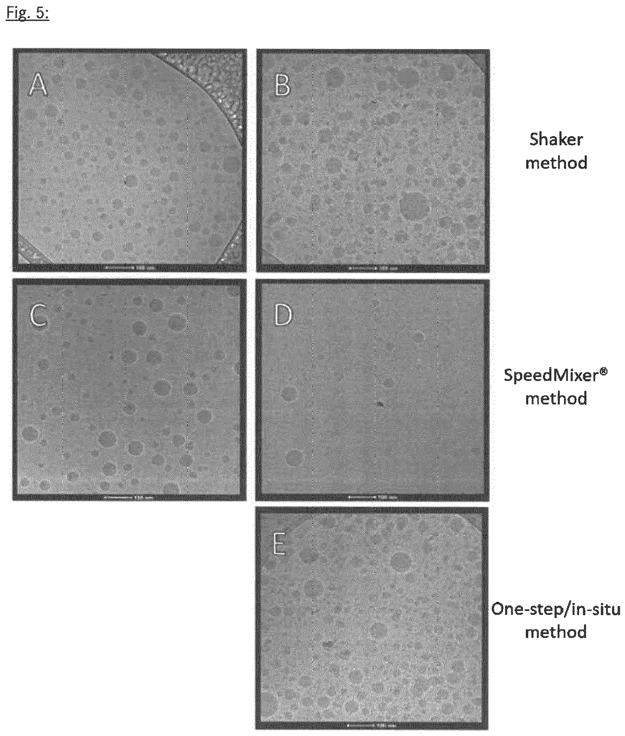

- Fig. 5:: Representative Cryo-TEM images of untreated PCBA-nanoparticulate systems (A, C) and nanoparticulate systems embedded into the gelatin matrix (B, C) produced by different manufacturing methods. E represents the one-step/in-situ process and therefore has no reference nanoparticulate system suspension. All samples contain 0.5% by mass of SDS as a surfactant (scale bar = 100 nm).

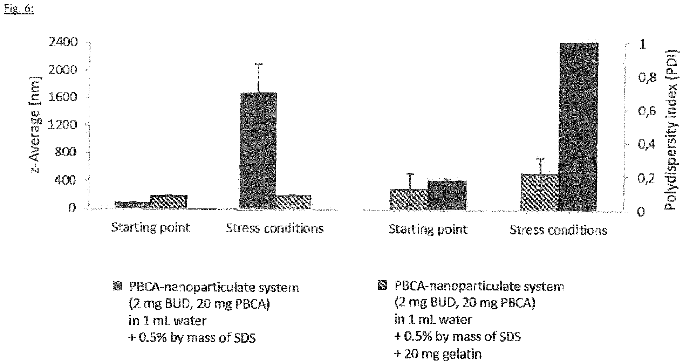

- Fig. 6:: Conventional PBCA-nanoparticulate system formulation under stress conditions compared to PBCA-nanoparticulate systems embedded in a gelatin matrix. The average particle size (left) and the polydispersity index (right) of the formulations were determined using DLS. The stress conditions were applied by using a modified freeze-thaw cycle test. Test conditions: temperature interval of 2.0 to 40.0°C for 15 min over 3 cycles under shaking at 420 rpm. The gelatin used for the test was a gelatin having a Bloom number of 260.

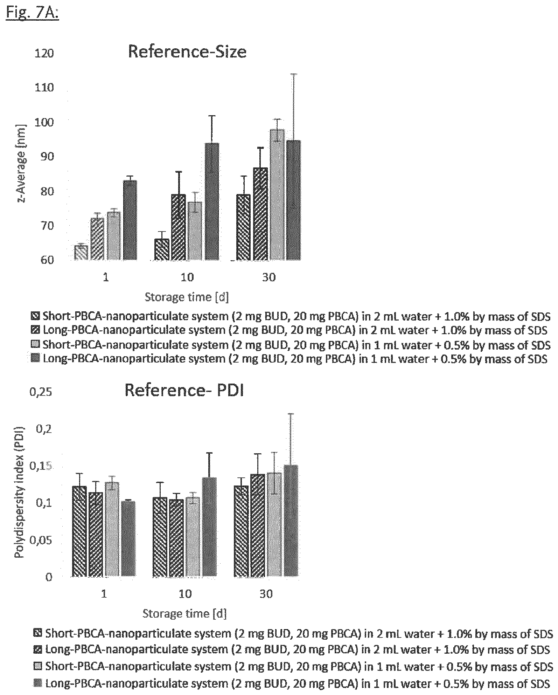

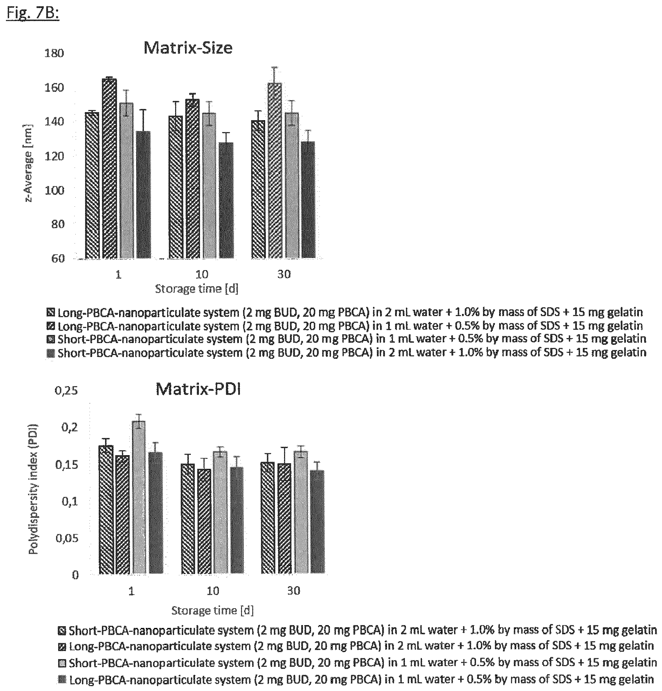

- Figs. 7A/7B:: Average particle size and polydispersity index of different nanoparticulate system formulations and their corresponding formulations, embedded in a gelatin matrix. The samples were examined on the day of manufacture, after 10 days, as well as after 30 days.

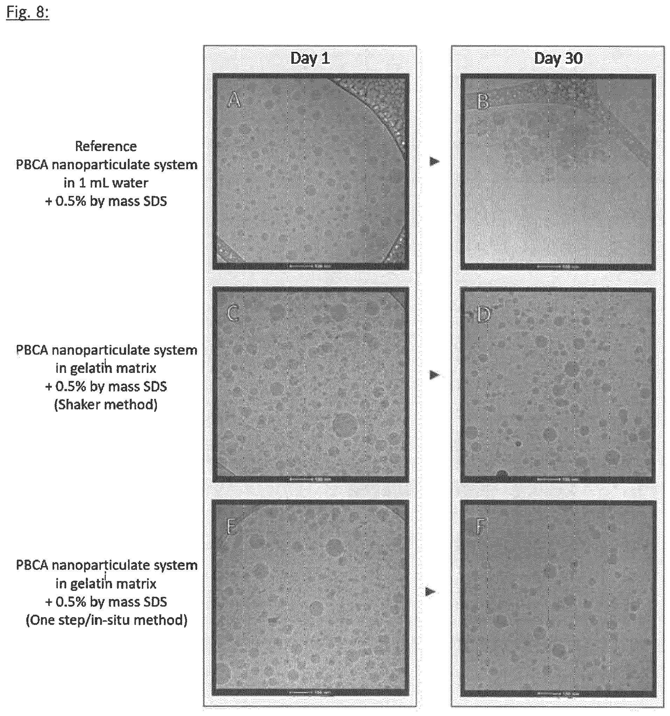

- Fig. 8:: Representative Cryo-TEM images of untreated drug-loaded PBCA nanoparticulate systems (A, B) and drug-loaded PBCA nanoparticulate systems in a gelatin matrix. The gelatin matrix was incorporated either during the manufacturing process (E, F) or afterwards (C, D). Images A, C and E show the day of manufacture. Images B, D, and F were taken after storage for 30 days (scale bar = 100 nm).

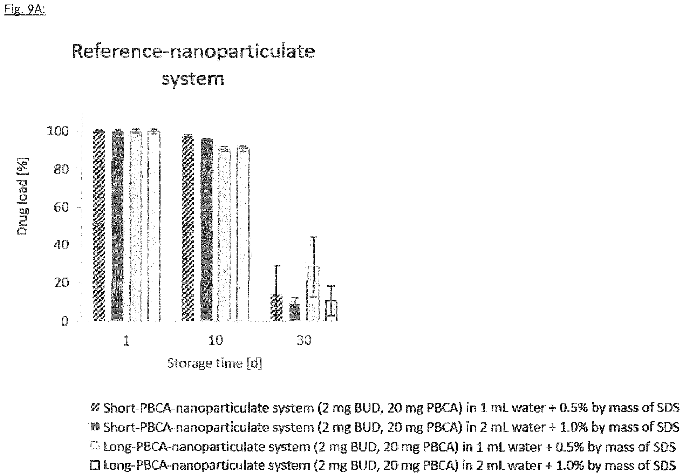

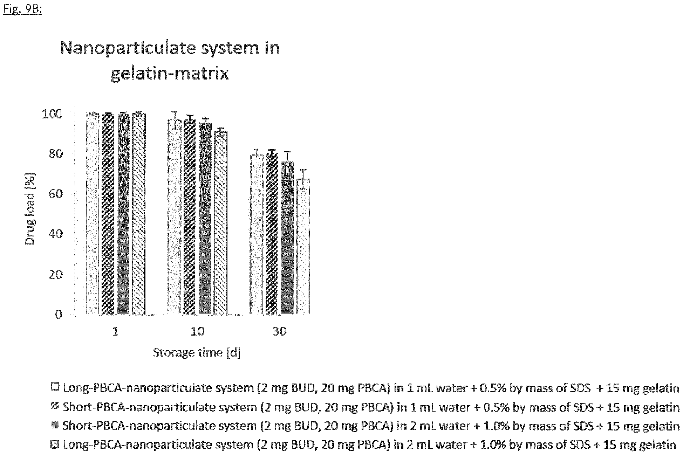

- Figs. 9A/9B:: Drug load of nanoparticulate system formulations over a storage period of 30 days. The tested formulations differ in terms of the polymer matrix and their particle concentration. Short-chain PBCA with an average number molecular weight (Mₙ) of ∼ 2000 g/mol or long-chain PBCA with an Mₙ of ∼ 20000 g/mol served as the basis of the matrix. In Figure 9A, the results of the reference versus, in Figure 9B, the corresponding formulation, which was embedded in a gelatin matrix. The samples were examined after 1, 10, and 30 days.

The present invention will be further illustrated in the following examples without being limited thereto.

### Examples

### Materials and methods:

### Materials

Poly (butyl cyanoacrylate) (PBCA) as the polymer was synthesized using monomers provided by TONG SHEN ENTERPRISE CO., LTD (Taiwan). Polyvinyl alcohol (PVA), sodium dodecyl sulfate (SDS) as the surfactant, chloroform as the organic solvent, and budesonide (BUD) as the active pharmaceutical ingredient were purchased from Sigma Aldrich, Germany. A selection of type B gelatins (Bloom number: 260 or 300) as the gelatin were obtained from Gelita AG, Germany.

### Characterization of the gel formation and gel melting temperature

Measurements of the sol-gel transition of the gel matrix were performed with a Haake MARS III^{™} rheometer (Thermo Scientific, Karlsruhe) using a parallel plate geometry with a diameter of 35 mm. Triplicates of samples containing 15 mg of gelatin in 1 mL demineralized water and either 3% by mass of PVA, 0.5% by mass of SDS, or no additive were studied. The gel samples were freshly prepared 17 hours before use and stored at 10.0°C. The samples were gently melted before application to the rheometer and rim of the measuring gap enclosed with a low-viscosity paraffin to avoid the loss of water. The sample was first cooled down to 4.0°C and then heated at a rate of 1.0°C/min. The rheometer was operated in a controlled shear deformation mode with 1% deformation and a frequency of 1 Hz while recording the shear stress amplitude. The dynamic viscoelastic moduli G' (storage modulus) and G" (loss modulus) were evaluated as a function of the temperature.

### Cryogenic transmission electron microscopy (Cryo-TEM)

Cryogenic transmission electron microscopy (Cryo-TEM) was used to characterize the morphology/shape, the average particle size, and the polydispersity index of the respective nanoparticulate systems in the different nanoparticulate system formulations. Therefore, 4 µl of the sample was placed on a carbon-coated support grid. After 3 seconds, the excess of sample solution was removed with a filter paper (Whatman, Germany). Subsequently, the carbon-coated support grid carrying the sample was frozen in liquid ethane at -180°C. The respective images were acquired using a CM200FEG microscope equipped with a high-resolution camera (4Kx 4K TemCam F416 CMOS). For all image acquisitions, the accelerating voltage was 200 kV and a low-dose system (20-30 e⁻/Å²) was used. Detailed images were taken at 50000 times magnification and overview images at 5000 times magnification.

### Production of drug-loaded poly (butyl cyanoacrylate) nanoparticulate systems by solvent evaporation

The drug-loaded nanoparticulate systems were produced using an oil-in-water solvent evaporation method (A.Ćurić et al., J. Pharm. Sci., 2015, 78, 121-131). 20 mg of PBCA and 5 mg of an active pharmaceutical ingredient, for example BUD, were dissolved in 1 mL of chloroform. The chloroform phase was added to the vehicle solution (0.5% by mass of SDS or 3% by mass of PVA in 1 mL demineralized water). Then both phases were emulsified for 5 min. using an ultrasonic rod (HTU SONI 130 G. Heinemann Ultraschall- und Labortechnik, Schwäbisch Gmünd, Deutschland) under ice cooling. The organic solvent was evaporated for 2 hours under the laboratory hood. The suspension was then filtered through a Minisart RC 15 filter (Sartorius Biotech, Göttingen, Germany) with a pore size of 0.22 µm to remove the non-encapsulated active pharmaceutical ingredient.

### Production of poly (butyl cyanoacrylate) nanoparticulate systems in a gelatin matrix

### Production of nanoparticulate systems using a heatable shaker

In case of shear-sensitive formulations, the prefabricated PBCA-nanoparticulate systems were incorporated into the gelatin matrix using a Thermomixer^{®} (R 5355 Eppendorf, Germany). Gelatin was added to the prefabricated nanoparticulate system suspension and left to swell for 15 minutes. The formulation was then shaken for about 30 minutes at 40.0°C until the gelatin was completely dissolved. The finished nanoparticulate system formulation was placed in the refrigerator to initiate the gel formation.

### Production of nanoparticulate systems using a SpeedMixer^{®}

In the case of thermolabile formulations, the nanoparticulate system suspension can be mixed directly with a prefabricated gelatin solution using a SpeedMixer^{®} (Hauschild GmbH & Co.KG, Germany). A gelatin solution with twice the target concentration was prepared and mixed with the finished nanoparticulate system suspension in an Eppendorf reaction vessel at 25.0°C in a ratio of 1:1. The suspension and the gelatin solution were then homogenized for 5 minutes in the SpeedMixer^{®}. The finished nanoparticulate system formulation was placed in the refrigerator to initiate the gel formation.

### Production of nanoparticulate systems using a one-step process

In this method the gelatin is directly added during the nanoparticulate system manufacturing process. The gelatin was dissolved at 40.0°C in the aqueous surfactant solution, which later forms the outer phase in the finished nanoparticulate system suspension. The surfactant/gelatin solution was then mixed with the chloroform phase, containing PBCA and the active pharmaceutical ingredient. The combined phases were then homogenized using a sonicator (HTU SONI 130, G. Heinemann Ultraschall- und Labortechnik, Schwäbisch Gmünd, Germany). Sonication was carried out for 4 minutes under ice cooling with 70% of the maximum amplitude and cycle 1. The resulting emulsion was then stirred for two hours, to ensure the complete evaporation of chloroform. The finished nanoparticulate system formulation was then placed in the refrigerator to initiate the gel formation.

### Particle stability under stress conditions

To verify the stabilizing effect of the gelatin matrix and to enable rapid testing of various compositions, the nanoparticulate system suspensions were exposed to stress conditions. The stress conditions were applied by using a modified freeze-thaw cycle test. The samples were first shaken in the Thermomixer^{®} (R 5355 Eppendorf, Germany) for 15 min at 2 C with 420 rpm. Subsequently, the samples were heated to 40.0°C and shaken again for 15 min. The freeze-thaw cycle was repeated 3 times. The particle size (z-average) of the nanoparticulate systems in the gel-matrix and their polydispersity index were examined by dynamic light scattering (DLS) using a Zetasizer Nano ZS (Malvern Panalytical Ltd, Malvern,UK).

### Long-term stability enhancement by the gelatin matrix

To compare the long-term stabilizing effect of the gelatin matrix, four different plain PBCA-nanoparticulate system formulations were compared with the corresponding gel matrix formulation. The poly (butyl cyanoacrylate) nanoparticulate system formulations were produced by the solvent evaporation method using 0.5% by mass of SDS as the surfactant. Two short-chain polymer formulations (Mₙ ∼ 2000 g/mol) and two long-chain polymer formulations (Mₙ ∼ 20000 g/mol) were compared. In each case, one formulation was produced with 10 mg/ml polymer, while the other one was produced with twice the amount of polymer and drug. Corresponding gelatin matrix formulations were produced by adding gel 15 mg/ml gelatin (Bloom number: 300) as described earlier. Triplets of each formulation were then aliquoted and stored at 5.0 ± 1.0°C. The samples were examined on day 1, 10 and 30 regarding their drug load, average particle size and polydispersity index. To determine the drug load, the nanoparticulate systems were first separated by Sephadex SpinTrap^{™} G-25 columns. Subsequently, the nanoparticulate systems were solved in acetonitrile and the drug load was determined using a photometer (Camspec M508 UV/Vis).

### Evaluations:

### Gel formation and gel melting temperatures

The temperature-dependent gel formation (sol-gel transition) and the inverse gel melting (gel-sol transition) of the samples were monitored by low deformation oscillatory rheology. The crossover of the storage modulus G' and the loss modulus G" were used to define the gel point upon cooling and the melting point for the subsequent heating.

The gel formation of the reference formulation containing 15 mg gelatin in 1 mL demineralized water started at 10.6°C and the corresponding gel started to melt at 28.0°C in the subsequent reheating step. The addition of 3% by mass of PVA to the gelatin solution slightly increased the gel point to 14.1°C, but did not show any effect on the melting point and the gel strength. In contrast, the physical gel properties were significantly altered by the addition of 0.5% by mass of SDS. The gel point was shifted to < 5.0°C and the gel formation was considerably delayed, indicating a different gelation mechanism as the result of interactions between the gelatin molecules and the functional groups of the ionic surfactant (R. Wüstneck et al., Col. Polym. Sci., 1988, 266, 1061-1067; C. Buron et al., Col. Polym. Sci., 2004, 282 (5), 446-453). The modified gel matrix already melted at 19.7°C owing to a substantial melting point depression.

The sol-gel and gel-sol transition temperatures of the gelatin matrix could be further modified by variation of the gelatin concentration and the gelatin/SDS ratio. A higher gelatin concentration leads to an increase in both transition temperatures and the stiffness of the gel. A similar alteration can be achieved by using different gelatin grades, e.g. lower or higher Bloom gelatins (data not shown). In any case, the addition of the ionic surfactant induced the necessary depression of the transition temperatures to achieve the desired performance profile: a gel formation temperature of 2.0 to 10.0°C and a gel melting temperature of 15.0 to 25.0°C. While the interplay between the gelatin grade, SDS and the gelatin concentration is mechanistically very complex, adjustments of the performance profile can be easily achieved by small variations of the gelatin concentration and/or the selection of the surfactant. In this context, the rheological data as depicted in Figure 4 provided evidence that the melting and gel formation temperatures of the gel matrix can be tailored to the specific needs of the application, such as the storage as a solid gel in the refrigerator while retaining a melting point just below or around room temperature.

### Morphology and average particle size of the poly (butyl cyanoacrylate) nanoparticulate systems embedded in the gelatin matrix

The Cryo-TEM images show the same nanoparticulate system shape for all tested samples. Round spheres consisting of a compact polymer matrix are the typical nanoparticulate systems obtained from the solvent evaporation method.

For the nanoparticulate systems embedded in the matrix using the shaker method, the evaluation of the Cryo-TEM images (B) showed an average particle size of 48.25 ± 23.73 nm (n = 200) and 49.11 ± 28.14 nm (n = 200) for the reference (A).

The high shear forces that arise when using the Speedmixer^{®} do not seem to have any influence on the morphology of the nanoparticulate system (D). The determined average particle sizes of the reference (C) were 66.54 ± 28.49 nm (n = 200) and 62.36 ± 32.27 nm (n = 100) in case of the embedded nanoparticulate systems (D).

Similar to the previous methods, the addition of gelatin during the nanoparticulate system production process has no influence on the morphology of the nanoparticulate system. The evaluation of the Cryo-TEM images showed an average particle size of 68.03 ± 28.16 nm (n = 200). A direct comparison with the results of the previous experiments (C, D), which were performed from the same batch of polymer, indicated no significant difference in the average particle size.

In this context, the Cryo-TEM images as depicted in Figure 5 provide evidence that independent of the embedding process the gelatin matrix does not affect the average particle size or morphology of the embedded nanoparticulated systems.

### Stability under stress conditions

To study the stability enhancing effect of the thermoreversible gelatin matrix under stress conditions, an untreated PBCA-nanoparticulate system formulation was compared to a PBCA-nanoparticulate system formulation embedded into a gelatin matrix (Bloom number: 260).

Under stress conditions, the reference suspension showed an increase in the average particle size from 94.1 ± 1.9 to 1678.6 ± 423.5 nm and the polydispersity index from 0.171 ± 0.013 to the maximum value of 1.0 (Figure 6). These results indicate strong aggregation and thus a loss of stability. In case of the embedded nanoparticulate systems, the average particle size determined by DLS before and after the stress test was 184.5 ± 7.4 nm and 188.4 ± 19.5 nm, respectively. The polydispersity index was 0.119 ± 0.094 before and 0.215 ± 0.095 after the test and thus within the respective standard deviation. The investigation clearly shows the stabilizing effects of the gelatin matrix on the nanoparticulate systems of the dispersion. The use of a gelatin having a Bloom number of 300 led to comparable results at a concentration of 15-25 mg gelatin per 1 mL demineralized water. Below 10 mg gelatin per 1 mL demineralized water, the stabilizing effect of the matrix on the nanoparticulate systems decreases.

### Long-term stability

All reference formulations show an increase in size and polydispersity within the examined time. On the other hand, the nanoparticulate systems that were embedded in the gelatin matrix do not show a significant change regarding average particle size or polydispersity index. All values for particle size or polydispersity lie within the standard deviation of the respective time points. The comparison of the different formulations (Figures 7A and 7B) shows that, as expected, the nanoparticulate systems without a matrix are less stable with increasing average particle size and concentration, because the collision rate of the nanoparticulate systems and thus the probability of the occurrence of agglomerates increases. This relationship has already been described in the literature not only for coarsely dispersed suspensions but also for nanoparticulate system formulations (M. Baalousha et al., Sci. Total Environ., 2009, 407(6), 2093-2101). On the other hand, no differences in stability could be observed between the nanoparticulate system formulations embedded in the gelatin matrix (at 8.0°C). The influence of the particle size and concentration and thus the collision rate and the gravitational force are minimized by the immobilization in the gelatin network.

The Cryo-TEM images (Figure 8) also confirm that the formulation without a gelatin matrix leads to the formation of agglomerates within 30 days (B). The samples containing nanoparticulate systems embedded in the matrix (D, F) do not show any morphological changes compared to the day of production (C, E). The stabilising effect is independent of the method used for production. The determination of the average particle size based on the Cryo-TEM image also confirms that the embedding of the nanoparticulate systems in the gelatin matrix leads to a significant improvement in stability, when stored for 30 days. The nanoparticulate systems embedded in the gelatin matrix by the shaker method had an average particle size of 49.29 ± 27.9 nm (n = 200) at the beginning of the investigation. After 30 days the average particle size was 51.37 ± 24.74 nm (n = 200). This implies that the average particle size of the nanoparticulate systems before and after 30 days storage do not differ significantly from each other (p = 0.719). When gelatin was added during production (in-situ method of producing), the nanoparticulate systems had an average particle size of 68.03 ± 28.16 nm at the beginning and 67.27 ± 29.62 nm at the end of the investigation (n = 200) (p = 0.259).

Regarding the drug load (Figures 9A and 9B), the reference nanoparticulate systems, did not show a significant decrease within the first 24 hours. After 10 days the amount of active pharmaceutical ingredient dropped to 90-97% of the initial value, depending on the formulation. On the last day of the investigation the active pharmaceutical ingredient content of these formulations was at 9-28%. The individual measurements after 30 days show a large inconsistency within each sample group. While the nanoparticulate systems with a gelatin matrix have a similar active pharmaceutical ingredient content to the reference nanoparticulate systems after 10 days, a significantly higher active pharmaceutical ingredient content of all formulations compared to the reference can be seen after 30 days. Depending on the formulation, the content is still around 67-80% of the initial value. In addition, the standard deviation after 30 days is significantly lower compared to the reference.

In view of the above, the present invention provides a newly developed aqueous gelatin-based gel matrix to stabilize a wide range of colloidal drug carriers while maintaining the benefits of a liquid formulation for medical application. This performance is achieved by a controlled modulation of the gel formation temperature and the gel melting temperature of the gel matrix. The physical properties of the gel matrix are modulated by the gelatin content and the addition of a surfactant and can be adapted to the specific needs of the application. It could be demonstrated that a gel matrix with e.g. 15 mg gelatin and 0.5% by mass of SDS in 1 mL demineralized water provides an effective and thermoreversible carrier matrix for any kind of nanoparticulate system. The matrix can be stored easily as solid and stable gel at low temperatures (e.g. at 2.0 to 10.0°C in a refrigerator) and quickly reliquefied at room temperature without the need of any technical equipment. This allows the storage of nanoparticulate systems in aqueous dispersion over months due to the immobilization of the nanoparticulate systems in the solid gel.

Neither the embedding process nor the thermally induced liquefaction affects the nanoparticulate system integrity. Long-term storage experiments over a period of 30 days showed significant advantages of the gel matrix formulation regarding average particle size, polydispersity index and drug load. In addition to the embedding of pre-produced nanoparticulate systems into the gel matrix in separate processes, it could be demonstrated that the gel matrix and the nanoparticulate systems can be produced in a one-step/in-situ process. This allows an integration of this approach into already existing production processes, which can reduce costs and effort in development and production.

The embedding of nanoparticulate systems in a gel matrix carrier provides a number of advantages compared to the established stabilization methods. In contrast to freeze-dried products there is no need for a redispersion prior to use. This reduces the risk of incorrect application and can help to improve dosing accuracy. Outside of a refrigerator the matrix liquefies at room temperature within minutes. In case of a rectal application, such as for the treatment of *ulcerative colitis,* gelatin can enhance the distribution and release behaviour of the nanoparticulate systems as well as improve the wound healing of the damaged mucosa (F. R. Diniz et al., Nanomaterials, 2020, 10(2), 390). And most important, the gel matrix carrier enables sufficient long-term storage of nanoparticulate systems and hence marketable drug products with the benefits of a liquid formulation.

## Claims

1. A thermoreversible sol-gel composition for stability enhancement and ready-to-use liquid application of nanoparticulate systems, comprising:
0.5 to 15.0% by mass of a gelatin having a Bloom number of 60 to 325,
a nanoparticulate system comprising at least one selected from the group consisting of a biological polymer, a synthetic polymer, a lipid, a metal core, and a silica core,
a surfactant, and
a biocompatible solvent essentially comprising water,
wherein the mass ratio [gelatin]/[surfactant(s)] is 0.5 to 10.0.

2. The thermoreversible sol-gel composition according to claim 1, wherein the gelatin is at least one selected from the group consisting of type A gelatins and type B gelatins.

3. The thermoreversible sol-gel composition according to claim 1 or 2, wherein the gelatin has a Bloom number of 260 to 300.

4. The thermoreversible sol-gel composition according to any one of claims 1 to 3, wherein the biological and/or synthetic polymer is at least one selected from the group consisting of a poly (meth)acrylate, a polyalkylacrylate, a polyether, a polyester, a polyketone, a polyolefin, a polyurethane, a polyamide, a polyamine, a polyurea, a polysiloxane, a polyvinylidene, an oligosaccharide, a polysaccharide, an oligopeptide, a polypeptide, an oligonucleotide, derivatives thereof, and copolymers thereof.

5. The thermoreversible sol-gel composition according to any one of claims 1 to 4, wherein the biological and/or synthetic polymer has a number average molecular weight (Mₙ) of 500 to 500000 g/mol.

6. The thermoreversible sol-gel composition according to any one of claims 1 to 5, wherein the nanoparticulate system has an average particle size of 1 to 500 nm.

7. The thermoreversible sol-gel composition according to any one of claims 1 to 6, comprising 0.1 to 5.0% by mass of the nanoparticulate system.

8. The thermoreversible sol-gel composition according to any one of claims 1 to 7, wherein the surfactant is at least one selected from the group consisting of an anionic surfactant, a cationic surfactant, a non-ionic surfactant, an amphoteric surfactant, and mixtures thereof.

9. The thermoreversible sol-gel composition according to any one of claims 1 to 8, wherein the mass ratio [gelatin]/[surfactant(s)] is 1.0 to 8.0.

10. A pharmaceutical composition, comprising the thermoreversible sol-gel composition for stability enhancement and ready-to-use liquid application of nanoparticulate systems according to any one of claims 1 to 9, wherein the nanoparticulate system further comprises an active pharmaceutical ingredient.

11. An in-situ method of producing the thermoreversible sol-gel composition according to any one of claims 1 to 9, wherein the nanoparticulate system comprises a biological polymer and/or a synthetic polymer, comprising:
providing a polymer solution comprising a biological polymer and/or a synthetic polymer dissolved in an organic solvent;
providing a gelatin-surfactant solution comprising a biocompatible solvent essentially comprising water, 0.5 to 15.0% by mass of a gelatin having a Bloom number of 60 to 325, and a surfactant, wherein the mass ratio [gelatin]/[surfactant(s)] is 0.5 to 10.0;
mixing the polymer solution with the gelatin-surfactant solution by ultra-sonification to form a gelatin-surfactant-polymer emulsion;
after said mixing, removing the organic solvent by evaporation to form a gelatin-nanoparticulate system formulation; and
after said evaporation, cooling the gelatin-nanoparticulate system formulation to a temperature of 2.0 to 10.0°C.

12. The method of producing according to claim 11, wherein the organic solvent is at least one selected from the group consisting of an alcohol, an ester, an ether, a ketone, a substituted or unsubstituted linear hydrocarbon, a substituted or unsubstituted cyclic hydrocarbon, and a mixture thereof.

13. The method of producing according to claim 11 or 12, wherein the polymer solution further comprises an active pharmaceutical ingredient dissolved in said organic solvent.

14. The thermoreversible sol-gel composition according to any one of claims 1 to 9 for use in medicine, wherein the nanoparticulate system further comprises an active pharmaceutical ingredient.

15. The thermoreversible sol-gel composition according to any one of claims 1 to 9 for use in treating *ulcerative colitis,*
wherein the nanoparticulate system further comprises budesonide in form of a nanocrystal formulation.
